# EUROPEAN PATENT APPLICATION

(11) **EP 1 293 179 A1**
(43) Date of publication of application: **19.03.2003**
(21) Application number: 02256320.9
(22) Date of filing: 12.09.2002
(51) Int. Cl.: A61F 2/36

(54) **Resilient thimble for ball head of prosthetic joint**

(30) Priority: 14.09.2001 GB 0122295
(71) Applicant: BENOIST GIRARD SAS, 14201 Hérouville-Saint-Clair Cédex (FR)
(72) Inventor: Cueille, Christophe, 14210 Missy (FR); Raugel, Patrick, 37530 Chargé (FR)
(74) Representative: Bridge-Butler, Alan James

(57) **Abstract**

A head component (1) for use in a prosthetic joint comprising a body (2) adapted to engage a natural or a prosthetic cup component and a resilient thimble (4) located therein adapted to engage the head spigot (18) of a prosthetic stem component with which it is to be used.

## Description

This invention relates to the ball head of a prosthetic joint particularly, but not exclusively, for use in a replacement hip joint, a prosthesis incorporating such a ball head, and a method of assembly thereof.

The Exeter and Charnley type femoral prostheses are well known and comprise a stem for implantation into the medullary canal and a neck at the proximal end which carried a ball head portion or head spigot for co-operation with a ball head component. With the Exeter design the ball head is dimensioned to co-operate with an acetabular cup prosthesis implanted into the acetabulum. With the Charnley design the ball head is sometimes dimensioned to co-operate with the natural acetabulum.

It is common for said femoral prostheses to be provided with a modular head rather than an integral ball head portion, because the stem can be more readily implanted into a medullary canal without the ball head attached. Further, if revision surgery is required to correct a failing ball head it can be removed from the spigot and replaced without removing the stem from the medullary canal. This reduction in surgery is beneficial for patients, particularly the elderly.

It is also known for ball heads to be constructed from a ceramics material as it provides a particularly suitable bearing surface. However, ball heads of this type are relatively brittle and can be damaged when subjected to loading of the joint by the patient after surgery, for example during walking. In these circumstances the ball head needs to be replaced in revision surgery, which is bad for patients, particularly the elderly.

It is further known for the head spigots of femoral stem components to suffer damage in the form of scratches. This can occur during fitting or removal of the ball head and during normal loading of the joint if the ball head is not securely fitted to the head spigot and makes minute movements thereon. It can also occur as the result of ball head failure. Unfortunately, a new ceramics ball head cannot be fitted onto a damaged head spigot. Scratches on the surface of a head spigot create ridges which can fracture a ceramics ball head during fitting or joint loading, leading to prosthesis failure. If it is found during revision surgery that the head spigot has also suffered damage for whatever reason, the femoral stem component has to be replaced. This significantly extends surgery time, which can be harmful to patients, particularly the elderly.

Therefore, it is proposed that by manufacturing a ceramic ball head with a relatively resilient and deformable thimble located therein, some of these problems can be overcome.

A thimble component made of a relatively resilient and deformable material such as titanium alloy can perform two functions. Firstly, it can absorb the defects of a damaged head spigot, so an imperfect femoral stem component would not need to be replaced during revision surgery.

Further, the resilient properties of the thimble can help to prevent structural damage being caused to the ceramics ball head by the less resilient head spigot during fitting and removal of the ball head and during loading of the joint. In addition the thimble can help to prevent damage being caused to the head spigot if the ball head is not securely fitted thereon and makes minute movements during loading.

Therefore, according to the present invention a head component for use in a prosthetic joint comprises a body adapted to engage a natural or a prosthetic cup component and a resilient thimble located therein adapted to engage the head spigot of a prosthetic stem component with which it is to be used.

The invention also includes a prosthesis comprising a stem and a neck carrying a head spigot which is engaged with a ball head component be means of a resilient thimble component located therein, which is adapted the engage said head spigot. Said ball head component being adapted to engage a natural or a prosthetic cup component.

The invention can be performed in various ways but one embodiment will now be described by way of example and with reference to the accompanying drawings in which:
Figure 1 is a cross-sectional side view of a ball head according to the present invention;
Figure 2 is a cross-sectional top view of a traditional ceramics ball head fitted to a damaged head spigot;
Figure 3 is a cross-sectional top view of a ceramics ball head according to the present invention fitted to a damaged head spigot;
Figure 4 is a side view of the ball head in Figure 1 being fitted onto the head spigot of a femoral prosthesis located in the medullary canal, with the ball head and the femoral prosthesis shown in cross section; and
Figure 5 is a cross sectional side view of a total hip prosthesis according to the present invention.

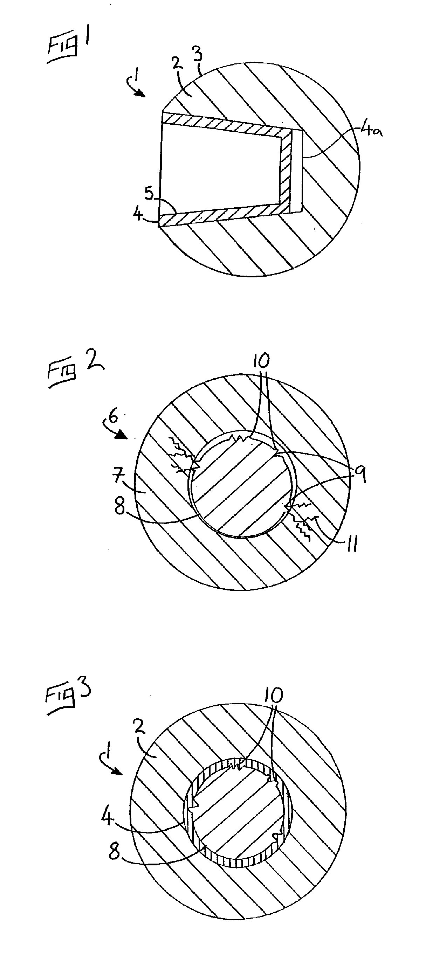

As shown in Figure 1 a ball head 1 for a femoral prosthesis has a body 2 formed from a ceramics material, for example alumina. The body 2 has a smooth bearing surface 3. The ball head 1 is provided with a thimble 4 constructed from a relatively resilient and deformable material, for example titanium. The thimble 4 is secured inside said body 2 by means of a preloading procedure during construction. A void 4a is left between the thimble 4 and the body 2. The inner surface of the thimble 5 is formed as a Morse tapered socket.

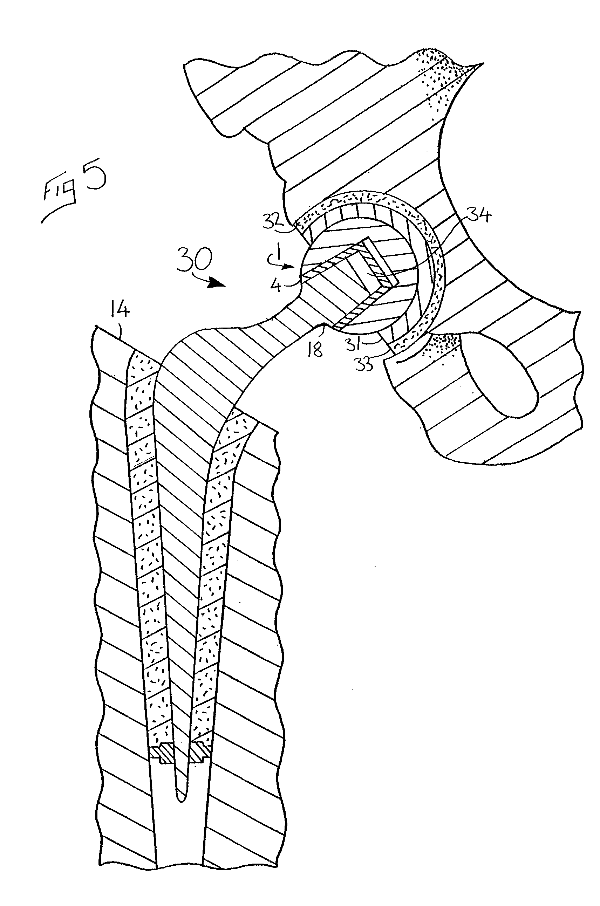

The ball head 1 and the resilient thimble 4 can be assembled together prior to assembly to a femoral stem component 12 (Figure 4). The head 1 and thimble 4 are preloaded under a force between 400N and 800N.

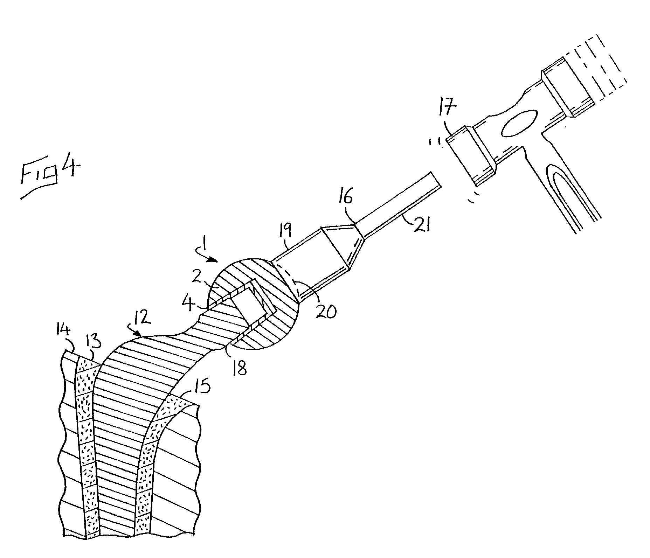

As shown in Figure 2 a traditional ball head 6 has a body 7 formed from a ceramics material, but it is not provided with an inner thimble. The ball head 6 has been fitted onto a head spigot 8, which has suffered damage in the form of vertical scratches 9 (shown here in exaggerated size). The material displaced by the scratches 9 has formed ridges 10. The ridges 10 prevent the ball head 6 from making full contact with the head spigot 8. As a result of the partial contact the body 7 is subjected to extreme stress during loading. The leads to fractures 11, and subsequent ball head failure.

Figure 3 shows the ball head 1 as shown in Figure 1, fitted onto the damaged head spigot 8 as shown in Figure 2. The thimble 4 has absorbed the shape of the ridges 10, and made full contact with the head spigot 8. As a result of the deformation of the thimble 4 the body 2 is subjected to equal stresses during loading and no fractures occur.

In Figure 4 ball head 1 is in the process of being attached to femoral stem component 12 which is already implanted in the medullary canal 13 of the femur 14 by means of cement 15. (A collarless Exeter type femoral stem component is shown here, but it will be appreciated that any known type of femoral stem component which features a head spigot can be used.) Reduction handle 16 and surgical hammering means 17 are used to secure the ball head 1 to the undamaged head spigot 18 of the femoral stem component 12. The reduction handle 16 has a head section 19 which is made from a plastics material, and features a hemispherical impaction end 20 for co-operation with the ball head 1, and a body section 21 adapted to co-operate with the surgical hammering means 17. The thimble 4 acts as a resilient damping means between the body of the ball head 2 and the head spigot 18 when impact pressure is applied to the body of the ball head 2 via the hammering means 17 and the reduction handle 16, which helps to prevent ball head failure during surgery.

In Figure 5 complete hip prosthesis 30 comprises a ball head 1 fully secured to a femoral stem component 12, and an acetabular cup component 31 which is implanted into the acetabulum 32 by means of cement 33. Void 34 is left between the head spigot 18 and the thimble 4. During normal gait the ball head 1 is subjected to the known multiple loads which can cause fractures to traditional ball heads. However, thimble 4 acts as a resilient dampening means between the femur 14 and the acetabulum 32 during normal gait and prevents the multiple loads resulting in ball head failure.

Thus this invention reduces the chance of ball head failure due to a damaged head spigot during revision surgery and during loading of the joint thanks to the deformable properties of the thimble. This prevents the need to replace prostheses which are found during revision surgery to have damaged or scratched head spigots. Moreover, the internal dimensions of the thimble can be varied as required for any particular head spigot. Thus for the same size of ball head the internal taper, length or diameter of the thimble can be varied.

Further, this invention provides a complete prosthesis comprising a stem portion provided with a head spigot, which is connected to a ball head by means of a resilient thimble. Such a prosthesis is less likely to suffer failure during loading of the joint because of the resilient properties of the thimble.

## Claims

1. A head component for use in a prosthetic joint comprising a body adapted to engage a natural or a prosthetic cup component and a resilient thimble located therein adapted to engage the head spigot of a prosthetic stem component with which it is to be used.

2. A head component as claimed in Claim 1 in which the body is ball shaped and provided with a smooth outer bearing surface.

3. A head component as claimed in Claim 2 in which the resilient thimble is located in a socket provided in the body.

4. A head component as claimed in Claim 3 in which a void is left between the top of the thimble and the base of the socket provided in the body.

5. A head component as claimed in Claim 4 in which the thimble is constructed from a more resilient material than the head spigot of the prosthetic stem component.

6. A head component as claimed in Claim 5 in which the thimble is constructed from a metal.

7. A head component as claimed in Claim 6 in which the thimble is constructed from titanium or a titanium alloy.

8. A head component as claimed in any of the above Claims in which the body is constructed from a ceramics material.

9. A head component as claimed in Claim 8 in which the body is constructed from alumina.

10. A head component as claimed in any of the above Claims in which the thimble is provided with a Morse tapered socket adapted to engage the head spigot of a prosthetic stem component with which it is to be used.

11. A head component as claimed in any one of the above Claims in which the thimble and body are assembled together and preloaded under a pressure of between 400N and 800N.

12. A head component as claimed in any of the above Claims which is adapted for use with a replacement hip joint.

13. A prosthesis comprising a stem and a neck carrying a head spigot, which is engaged with a head component as set forth in any one of the preceding claims.

14. A prosthesis as claimed in Claim 13 in which the head spigot is damaged, and in which the thimble has absorbed the shape of the spigot and has made full contact with it's outer surface.

15. A method of assembling a head component as set forth in any one of the preceding Claims to the head spigot of a stem component already implanted into a bone canal including the steps:
i) Positioning the head component onto the head spigot
ii) Applying impact pressure to the head component by means of a reduction handle comprising a head section constructed from a plastics material and provided with a hemispherical impaction end for co-operation with the ball head, and a surgical hammering means.
